# EUROPÄISCHE PATENTSCHRIFT

(11) **EP 0 735 881 B1**
(45) Veröffentlichungstag und Bekanntmachung des Hinweises auf die Patenterteilung: **27.08.2003**
(21) Anmeldenummer: 95901345.9
(22) Anmeldetag: 24.11.1994
(51) Int. Cl.: A61K 31/57, A61K 31/565

(54) **MITTEL UND VERFAHREN ZUR HORMONALEN KONTRAZEPTION UND/ODER ZUR AKNEBEHANDLUNG**
MEANS AND METHOD FOR HORMONAL CONTRACEPTION AND/OR THE TREATMENT OF ACNE
AGENTS ET PROCEDE POUR LA CONTRACEPTION HORMONALE ET/OU POUR LE TRAITEMENT DE L'ACNE

(30) Priorität: 24.12.1993 DE 4344405
(43) Veröffentlichungstag der Anmeldung: 09.10.1996
(73) Patentinhaber: Ehrlich, Marika, Dr. med., D-55234 Framersheim (DE); Kuhl, Herbert, Prof. Dr., D-63741 Aschaffenburg (DE)
(72) Erfinder: Ehrlich, Marika, Dr. med., D-55234 Framersheim (DE); Kuhl, Herbert, Prof. Dr., D-63741 Aschaffenburg (DE)
(74) Vertreter: Goddar, Heinz J., Dr.
(86) Internationale Anmeldenummer: DE9401420
(87) Internationale Veröffentlichungsnummer: WO95017895

(56) Entgegenhaltungen:
- WO-A-90/04330
- WO-A-92/13539
- DE-A- 4 308 406
- DE-A- 4 313 926

## Beschreibung

Die Erfindung betrifft ein Mittel zur hormonalen Kontrazeption und/oder Aknebehandlung, mit zwei in einer Verpackungseinheit räumlich getrennt konfektionierten, zur zeitlich sequentiellen Verabreichung bestimmten Hormonkomponenten, die jeweils aus einer Anzahl räumlich getrennt und einzeln entnehmbar in der Verpackungseinheit untergebrachter Hormon-Tageseinheiten bestehen, wobei eine erste der Hormonkomponenten als hormonellen Wirkstoff im wesentlichen ausschließlich ein eine Störung der Follikelreifung bewirkendes Östrogenpräparat, die zweite Hormonkomponente hingegen in Kombination ein Östrogen- und in mindestens zur Ovulationshemmung ausreichender Dosierung ein Gestagenpräparat enthält und die Gesamtzahl der Hormontageseinheiten gleich der Gesamtzahl der Tage des gewünschten Zyklus ist, die erste Hormonkomponente 5 bis 14 und die zweite Hormonkomponente 23 bis 14 Tageseinheiten umfaßt und die Anzahl der Tageseinheiten der ersten Hormonkomponente geringer als die Anzahl der Tageseinheiten der zweiten Hormonkomponente ist.

Aus der DE-PS 41 04 385 sind ein ovulationshemmendes Mittel sowie ein Verfahren zur hormonalen Kontrazeption der vorstehend beschriebenen Art bekannt, bei denen vorzugsweise zur hormonalen Kontrazeption innerhalb des gewünschten Zyklus tageweise aufeinanderfolgend zunächst die Tageseinheiten der ersten Hormonkomponente und daran anschließend diejenigen der zweiten Hormonkomponente verabreicht werden, worauf sich unter Ausschluß von Einnahmepausen wiederum die erste der Tageseinheiten der ersten Hormonkomponente des nächstfolgenden Zyklus anschließt.

Das ovulationshemmende Mittel der gattungsgemäßen Art, bei dem das bei den bis dahin angewandten Typen von Ovulationshemmern übliche einnahmefreie Intervall von sechs oder auch sieben Tagen durch die durchgehende Einnahme der Östrogenkomponente, das heißt der Tageseinheiten der ersten Hormonkomponente, ersetzt wird, hat sich durchaus bewährt. Es hat sich allerdings gezeigt, daß dann, wenn die erste Hormonkomponente in möglichst niedriger Dosierung der Tageseinheiten eingesetzt wird, wie es günstig ist, um das gesundheitliche Risiko zu minimieren, beim erstmaligen Einsatz des Mittels die Follikelreifung zwar gestört, die Ovulation jedoch nicht zuverlässig verhindert wird. Diese geringere kontrazeptionelle Sicherheit im ersten Einnahmezyklus, verglichen mit den folgenden Einnahmezyklen, beruht darauf, daß die neue Follikelreifung bereits im letzten Teil der Lutealphase des vorhergehenden ovulatorischen Zyklus beginnt, wenn das endogene Estradiol und das endogene Progesteron abfallen.

Bei der Erstanwendung eines Ovulationshemmers herkömmlicher Art, der ein einnahmefreies Intervall vorsieht, wobei mit der Einnahme der Tabletten normalerweise etwa am fünften Tag nach Beginn der Menstruation angefangen wird, läßt sich die kontrazeptive Sicherheit dadurch erhöhen, daß bei der Erstanwendung bereits am ersten Tag der Menstruation der Einnahme begonnen wird (Martindale 1993, S. 1177). Durch die Applikation der Östrogen/Gestagen-Kombination zu diesem frühen Zeitpunkt wird die Ovulation bei den herkömmlichen Ovulationshemmern hierdurch zuverlässig verhindert, wobei in Kauf genommen wird, daß der erste Einnahmezyklus verkürzt ist, das heißt also, wobei sich eine Zykluslänge bis zum Beginn der nächsten Entzugsblutung von etwa 24 Tagen ergibt. Aus demselben Grund wurde auch bei herkömmlichen Präparaten bereits empfohlen, bei Umstellung von einem hochdosierten Ovulationshemmer auf einen niedriger dosierten Ovulationshemmer das einnahmefreie Intervall zu verkürzen oder im ersten Einnahmezyklus ganz wegzulassen (s. a. a. o.).

Da in einem normalen ovulatorischen Zyklus also die Follikelreifung bereits im letzten Drittel der Lutealphase (vor der Menstruation) beginnt, läßt sich beim ovulationshemmenden Mittel der gattungsgemäßen Art eine zuverlässige Kontrazeption bei Ersteinnahme selbst dann bei besonders niedrig dosierten Tageseinheiten der ersten Hormonkomponente nicht zuverlässig gewährleisten, wenn man mit der Einnahme der ersten Hormonkomponente am ersten Tag der Menstruation beginnt, zumal dann der erste Einnahmezyklus bis zur folgenden Entzugsblutung auf bis zu etwa 30 Tage verlängert würde.

Aus der DE-PS 43 08 406 (nicht vorveröffentlicht) ist bereits ein ovulationshemmends Mittel in Form eines Kombinationspräparates zur Kontrazeption bekannt, bei dem zumindest eine sowohl östrogen- als auch gestagenhaltige Hormonkomponente vorgesehen ist, bei der die Tageseinheiten sowohl ein biogenes Östrogen als auch ein synthetisches Östrogen enthalten. Die Erfindung bezieht sich nicht auf derartige Präparate.

WO90/04330 offenbart ein Verfahren zur Kontrazeption, welches ein zweistufiges Protokoll umfaßt. In der ersten Stufe wird einer Frau eine Östrogenkomponente in einer ersten Zusammensetzung täglich als die einzige kontrazeptive Verbindung von etwa Tag 2 bis etwa Tag 7 ihres Menstruationszyklusses verabreicht, wobei Tag 1 der erste Tag der Menses ist. Die zweite Stufe des Protokolls schließt sich unmittelbar daran an und während dieser Zeit wird der Frau täglich wenigstens eine Folgezusammensetzung verabreicht, die Progestin enthält. Die Folgezusammensetzung kann ein Progestin als den einzigen kontrazeptiven Bestandteil enthalten oder eine Kombination aus einer Östrogen-Verbindung mit einem Progestin in verschiedenen Gewichtsverhältnissen sein.

WO92/13539 schließlich betrifft ein ovulationshemmendes Mittel zur hormonalen Kontrazeption mit zwei in einer Verpackungseinheit räumlich getrennt konfektionierten, zur zeitlich sequentiellen oralen Verabreichung bestimmten Hormonkomponenten, die jeweils aus einer Anzahl räumlich getrennter und einzel entnehmbarer in der Verpackungseinheit untergebrachter Hormon-Tageseinheiten bestehen, wobei eine erste der Hormonkomponenten als hormonellen Wirkstoff im wesentlichen ausschließlich ein eine Störung der Follikelreifung bewirkendes Östrogenpräparat, die zweite Hormonkomponente hingegen in Kombinationen ein Östrogen - und in mindestens zur Ovulationshemmung ausreichender Dosierung ein Gestagen-Präparat enthält, wobei die Gesamtzahl der Hormon-Tageseinheiten gleich der Gesamtzahl der Tage des gewünschten Zyklus ist, die erste Hormonkomponente 5-14 und die zweite Hormonkomponente 23-14 Tageseinheiten umfaßt und die Anzahl der Tageseinheiten der ersten Hormonkomponente geringer als die Anzahl der Tageseinheiten der zweiten Hormonkomponente ist.

Der Erfindung liegt die Aufgabe zugrunde, das ovulationshemmende Mittel sowie das Verfahren der gattungsgemäßen Art dahingehend weiterzubilden, daß sich auch bei Ersteinnahme selbst bei Verwendung sehr niedrig dosierter Tageseinheiten der ersten Hormonkomponente eine hohe kontrazeptionelle Sicherheit ergibt.

Erfindungsgemäß wird diese Aufgabe in Weiterbildung des ovulationshemmenden Mittels der gattungsgemäßen Art gelöst durch eine derartige Anordnung der Tageseinheiten in der Verpackungseinheit, daß die Tageseinheiten der zweiten Hormonkomponente als zuerst und die Tageseinheiten der ersten Hormonkomponente als daran anschließend einzunehmen charakterisiert sind, wobei die Tageseinheiten der zweiten Hormonkomponente nicht die Kombination eines biogenen Östrogens und eines synthetischen Östrogens enthalten.

Weitere Ausführungsformen ergeben sich aus den Unteransprüchen.

Der Erfindung liegt die überraschende Erkenntnis zugrunde, daß es gelingt, auch bei ovulationshemmenden Mitteln der gattungsgemäßen Art eine hohe kontrazeptive Sicherheit im ersten Anwendungszyklus zu erreichen, wenn mit der Einnahme der zweiten Hormonkomponente, also der Östrogen/Gestagen-Komponente, am ersten Tag der Menstruation begonnen wird. In entsprechender Weise kann auch bei Umstellung von einem höher dosierten Präparat herkömmlicher Art auf ovulationshemmende Mittel der gattungsgemäßen Art verfahren werden, also sofortige Einnahme der Tageseinheiten der zweiten Hormonkomponente im Anschluß an die letzte Östrogen/ Gestagen-Tablette des herkömmlichen Präparates, obwohl dies eigentlich nicht notwendig wäre.

Das erfindungsgemäße Verfahren läßt sich unter Verwendung einer üblichen Packung (z. B. 7 Tageseinheiten der ersten Hormonkomponente und 21 Tageseinheiten der zweiten Hormon-komponente) der gattungsgemäßen Art dadurch durchführen, daß im ersten Einnahmezyklus die sieben Tageseinheiten bzw. Tabletten der ersten Hormonkomponente verworfen werden, so daß man also sofort am ersten Menstruationstag mit der Einnahme der ersten Tageseinheit der zweiten Hormonkomponente beginnt. Daran anschließend erfolgt die Einnahme nach dem üblichen Schema.

Andererseits kann aber auch so vorgegangen werden, daß eine Verpackungseinheit verwendet wird, in der die zweite Hormonkomponente den Einnahmetagen 1 bis 21, bei einem gewünschten Gesamtzyklus von 28 Tagen, und die erste Hormonkomponente den Einnahmetagen 22 bis 28 zugeordnet wird, woraufhin dann die nächste Verpackungseinheit, beginnend mit der zweiten Hormonkomponente, anschließt, etc..

Eine weitere Alternative besteht darin, eine Verpackungseinheit zu verwenden, bei der die Ziffern der Einnahmetage durch den Benutzer eingestellt werden können, so daß der erste Einnahmetag entweder auf die erste Tageseinheit der ersten Hormonkomponente oder aber auf die erste Tageseinheit der zweiten Hormonkomponente fällt, wobei ggf. eine runde Packungsform verwendet werden kann, wie sie z. B. beim handelsüblichen Präparat "Trisequens" Verwendung findet.

Die erste Hormonkomponente und die zweite Hormonkomponente können bei dem ovulationshemmenden Mittel nach der Erfindung im übrigen entsprechend der DE-PS 41 04 385 zusammengesetzt sein, auf welche zur weiteren Erläuterung insoweit verwiesen wird.

Zu der bei der Erfindung ggf., wenn auch nicht vorzugsweise, vorgesehenen Möglichkeit der transdermalen Verabreichung mindestens einer der Hormonkomponenten ist zu bemerken, daß es aus Brit. Med. J. 297 (1988), 900-901 bereits bekannt war, eine Ovulationshemmung durch transdermale Applikation von täglich 200 µg Estradiol - mit Hilfe von Pflastern, die alle drei Tage gewechselt werden - zu bewirken. Die Ovulationshemmung kommt dabei durch die Wirkung des permament erhöhten Estradiolspiegels zustande, ohne daß ein Gestagen verabreicht wird. Zur Vermeidung einer Endometriumhyperplasie kann dabei in jedem Zyklus ein Gestagen über 7 bis 10 Tage zusätzlich gegeben werden, welches jedoch wegen der Kürze der Anwendung keinen wesentlichen Beitrag zur Kontrazeption leistet. Die beschriebene Methode ist bei jungen Frauen weniger zuverlässig und sieht, anders als die Erfindung, nicht den Einsatz einer Hormonkomponente vor, bei der ein Gestagenpräparat in mindestens zur Ovulationshemmung ausreichender Dosierung vorhanden ist.

Nachstehend sind Ausführungsbeispiele der Erfindung beschrieben.

### Beispiel 1:

Zur ovulationshemmenden Behandlung wurde ein Sequenzpräparat verwendet, welches 21 Tageseinheiten mit jeweils 2 mg Östradiol und 1 mg Norethisteronacetat sowie 7 Tageseinheiten mit jeweils 2 mg Östradiol enthielt. Die erste Tageseinheit mit 2 mg Östradiol und 1 mg Norethisteronacetat wurde am ersten Tag der Menstruation bei Erstverabreichung genommen, woraufhin die weiteren 20 Tageseinheiten mit jeweils 2 mg Östradiol und 1 mg Norethisteronacetat und daran anschließend die 7 Tageseinheiten mit jeweils 2 mg Östradiol verabreicht wurden, mit unmittelbarem Anschluß der ersten der 21 Tageseinheiten mit jeweils 2 mg Östradiol und 1 mg Norethisteronacetat der nächstfolgenden Packung. Das Mittel wurde über 1 Jahr verabreicht und zeigte von Anfang an bei sehr guter kontrazeptiver Sicherheit praktisch keine Nebenwirkungen.

### Beispiel 2:

Es wurde ein ovulationshemmendes Mittel verwendet, welches 18 Tageseinheiten mit jeweils 20 µg Ethinylestradiol und 150 µg Levonorgestrel sowie 10 Tageseinheiten mit jeweils 20 µg Ethinylestradiol enthielt. Die Verabreichung entsprach Beispiel 1. Die Beobachtungen entsprachen ebenfalls dem Beispiel 1.

### Beispiel 3:

Zur ovulationshemmenden Behandlung wurde alle vier Wochen (jeweils am Tag 1) eine intramuskuläre Injektion von 50 mg Estradiolundecylat vorgenommen. Zusätzlich wurden von Tag 6 bis Tag 28 (23 Tageseinheiten) täglich 2 mg Cyproteronacetat oral eingenommen. Das Mittel zeigte bei guter kontrazeptiver Sicherheit praktisch keine Nebenwirkungen.

### Beispiel 4:

Zur ovulationshemmenden Behandlung wurden täglich 100 µg Estradiol transdermal - mit Hilfe eines Pflasters, das zweimal pro Woche gewechselt wurde - appliziert, d. h. am Tag 1, Tag 4, Tag 8, Tag 11 usw., dementsprechend alle 3,5 Tage. Zusätzlich wurden zwischen Tag 8 und Tag 28 täglich (21 Tageseinheiten) jeweils 150 µg Desogestrel verabreicht. Auch hierbei wurden keine nennenswerten Nebenwirkungen beobachtet, bei guter kontrazeptiver Sicherheit.

Die in der vorstehenden Beschreibung, in der Zeichung sowie in den Ansprüchen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Mittel zur hormonalen Kontrazeption und/oder Aknebehandlung, mit zwei in einer Verpackungseinheit räumlich getrennt konfektionierten, zur zeitlich sequentiellen Verabreichung bestimmten Hormonkomponenten, die jeweils aus einer Anzahl räumlich getrennt und einzeln entnehmbar in der Verpackungseinheit untergebrachter Hormon-Tageseinheiten bestehen, wobei eine erste der Hormonkomponenten als hormonellen Wirkstoff im wesentlichen ausschließlich ein eine Störung der Follikelreifung bewirkendes Östrogenpräparat, die zweite Hormonkomponente hingegen in Kombination ein Östrogen- und in mindestens zur Ovulationshemmung ausreichender Dosierung ein Gestagenpräparat enthält und die Gesamtzahl der Hormontageseinheiten gleich der Gesamtzahl der Tage des gewünschten Zyklus ist, die erste Hormonkomponente 5 bis 14 und die zweite Hormonkomponente 23 bis 14 Tageseinheiten umfaßt und die Anzahl der Tageseinheiten der ersten Hormonkomponente geringer als die Anzahl der Tageseinheiten der zweiten Hormonkomponente ist, **gekennzeichnet durch** eine derartige Anordnung der Tageseinheiten in der Verpackungseinheit, daß die Tageseinheiten der zweiten Hormonkomponente als zuerst und die Tageseinheiten der ersten Hormonkomponente als daran anschließend einzunehmen charakterisiert sind, wobei die Tageseinheiten der zweiten Hormonkomponente nicht die Kombination eines biogenen Östrogens und eines synthetischen Östrogens enthalten.

2. Mittel nach Anspruch 1, **dadurch gekennzeichnet, daß** die Gesamtzahl der Tageseinheiten fortlaufend nach Einnahmetagen, beginnend mit der Zuordnung der ersten Tageseinheit der zweiten Hormonkomponente zum ersten Einnahmetag, über den gesamten gewünschten Zyklus numeriert ist.

3. Mittel nach Anspruch 2, **gekennzeichnet durch** eine feste Numerierung der Tageseinheiten.

4. Mittel nach Anspruch 2, **gekennzeichnet durch** eine variable Numerierung der Tageseinheiten in der Weise, daß der erste Einnahmetag wahlweise der ersten Tageseinheit der ersten oder der zweiten Hormonkomponente zuschreibbar ist.

5. Mittel nach einem der vorangehenden Ansprüche, **dadurch gekennzeichnet, daß** mindestens eine der Hormonkomponenten mindestens teilweise zur oralen Verabreichung ausgebildet ist.

6. Mittel nach einem der vorangehenden Ansprüche, **dadurch gekennzeichnet, daß** mindestens eine der Hormonkomponenten mindestens teilweise zur transdermalen Verabreichung ausgebildet ist.

7. Mittel nach einem der vorangehenden Ansprüche, **dadurch gekennzeichnet, daß** mindestens einige der Tageseinheiten mindestens einer der Hormonkomponenten zu (jeweils) einer Depoteinheit zusammengefaßt sind.

8. Mittel nach einem der vorangehenden Ansprüche, **dadurch gekennzeichnet, daß** mindestens zwei Paare aus erster und zweiter Hormonkomponente in einer Verpackungseinheit räumlich getrennt konfektioniert sind.

9. Mittel nach Anspruch 7 und Anspruch 8, **gekennzeichnet durch** mindestens eine wenigstens zwei Hormonkomponentenpaare überlappende Depoteinheit.

10. Mittel nach einem der vorangehenden Ansprüche, **dadurch gekennzeichnet, daß** die Tageseinheiten der zweiten Hormonkomponente(n) einen stufenweise ansteigenden Östrogengehalt aufweisen.

## Claims

1. An agent for hormonal contraception and/or treatment of acne, comprising two hormone components for sequential administration, packed separately in a packaging unit and each comprising a number of spatially separated daily hormone units individually removable from the packaging unit, wherein a first hormone component constituting the active hormone substance consists substantially exclusively of an oestrogen preparation for disturbing the maturation of follicles whereas the second hormone component contains an oestrogen preparation in combination with a gestagen preparation in a dosage at least sufficient to inhibit ovulation and the total number of daily hormone units is equal to the total number of days in the desired cycle, the first hormone component contains 5 to 14 daily units and the second hormone component contains 23 to 14 daily units and the number of units of the first hormone component is less than the number of units of the second hormone component, **characterised by** an arrangement of daily units in the packaging unit such that the daily units of the second hormone component are specified as being taken first and the daily units of the first hormone component are specified as being taken afterwards, wherein the daily units of the second hormone component do not contain a biogenic oestrogen in combination with a synthetic oestrogen.

2. An agent according to claim 1, **characterised in that** the total number of daily units are numbered in succession in accordance with the day for ingestion over the entire desired cycle, beginning with the first daily unit of the second hormone component to be ingested on the first day.

3. An agent according to claim 2, **characterised by** a fixed number of the daily units.

4. An agent according to claim 2, **characterised by** variable numbering of the daily units in that the first day for ingestion is allocated to the first day for ingestion of either the first or the second hormone component as desired.

5. An agent according to any of the preceding claims, **characterised in that** at least one of the hormone components is intended at least partly for oral administration.

6. An agent according to any of the preceding claims, **characterised in that** at least one of the hormone components is designed at least partly for transdermal administration.

7. An agent according to any of the preceding claims, **characterised in that** at least some of the daily units of at least one of the hormone components are combined in a respective depot unit.

8. An agent according to any of the preceding claims, **characterised in that** at least two pairs of first and second hormone components are packed at separate places in a packaging unit.

9. An agent according to claim 7 and claim 8, **characterised by** at least one depot unit overlapping at least two pairs of hormone components.

10. An agent according to any of the preceding claims, **characterised in that** the daily units of the second hormone component or components comprise a stepwise increasing oestrogen content.

## Revendications

1. Moyen de contraception hormonale et / ou de traitement de l'acné, avec deux composants hormonaux, placés dans une unité de conditionnement en étant séparés matériellement, pour une administration séquentielle dans le temps de composants hormonaux déterminés, cette unité de conditionnement comprenant un certain nombre d'unités hormonales journalières, qui sont séparées matériellement et qui peuvent êtres administrées individuellement, où le premier des composants hormonaux est constitué d'une substance active horrnonale qui est une préparation d'esfrogènes dont l'effet est quasi exclusivement de perturber la maturation du follicule et le second composant hormonal contient, par contre, une combinaison d'une préparation estrogène et d'une préparation progestative dosée suffisamment pour avoir au moins un effet d'inhibition de l'ovulation, le nombre total d'unités hormonales journalières étant égal au nombre total du cycle souhaité, le premier composant hormonal étant présent dans 5 à 14 unités hormonales journalières et le second composant dans 23 à 14 unités journalières, le nombre d'unités journalières du premier composant hormonal étant plus petit que le nombre d'unités journalières du second composant hormonal, **caractérisé en ce que** le rangement des unités journalières dans l'unité de conditionnement est tel que les unités journalières du second composant hormonal sont prises d'abord et que les unités journalières du premier composant hormonal sont prises après, les unités journalières du second composant hormonal ne contenant pas une combinaison d'un estrogène biologique et d'un estrogène de synthèse.

2. Moyen selon la revendication 1, **caractérisé en ce que** toutes les unités journalières sont numérotées pour toute la durée souhaitée du cycle d'après le jour d'administration, l'administration de la première unité journalière du second composant correspondant au premier jour d'administration.

3. Moyen selon la revendication 2, caractérisé per une numérotation fixe des unités journalières.

4. Moyen selon la revendication 2, **caractérisé par** une numérotation des unités journalières qui est variable, en ce sens que le premier jour d'administration peut être attribué à la première unité journalière du premier composant hormonal ou à la première unité journalière du second composant horrnonal.

5. Moyen selon l'une des revendications précédentes, **caractérisé en ce qu'**au moins un des composants hormonaux est prévu pour être administré, au moins en partie, par voie orale.

6. Moyen selon l'une des revendications précédentes, **caractérisé en ce qu'**au moins un des composants hormonaux est prévu pour être administré, au moins en partie, par voie transcutanée.

7. Moyen selon l'une des revendications précédentes, **caractérisé en ce qu'**au moins une des unités journalières d'au moins un des composants hormonaux est réalisée sous la forme d'une unité à effet retard.

8. Moyen selon l'une des revendications précédentes, **caractérisé en ce qu'**au moins deux paires des premier et second composants hormonaux sont disposées dans une unité de conditionnement réalisée de manière matériellement séparée.

9. Moyen selon la revendication 7 et la revendication 8, **caractérisé par** au moins deux unités à effet retard correspondant à une paire de composants hormonaux.

10. Moyen selon l'une des revendications précédentes, **caractérisé en ce que** les unités journalières du second composant hormonal présentent une teneur en estrogène qui croit par étapes.
